Europäisches Patentamt

European Patent Office

Office européen des brevets

(11) Publication number: **0 152 485 B1**

# (12) EUROPEAN PATENT SPECIFICATION

(45) Date of publication of patent specification: **08.01.92**

(51) Int. Cl.⁵: **B01J 29/28**, C07C 2/66, C07C 15/02, C10G 45/64

(21) Application number: **84901099.6**

(22) Date of filing: **09.03.84**

(86) International application number: **PCT/JP84/00094**

(87) International publication number: **WO 84/03452 (13.09.84 84/22)**

(54) **BINDER-FREE ZEOLITE CATALYST, PROCESS FOR ITS PREPARATION, AND CATALYTIC REACTION USING SAME.**

(30) Priority: **09.03.83 JP 39721/83**

(43) Date of publication of application:
**28.08.85 Bulletin 85/35**

(45) Publication of the grant of the patent:
**08.01.92 Bulletin 92/02**

(84) Designated Contracting States:
**FR**

(56) References cited:
**EP-A- 0 101 232**

**PATENTS ABSTRACTS OF JAPAN, vol. 7, no. 131 (C-169) [1276], June 8, 1983; & JP - A - 58 45 111 (TOA NENRYO KOGYO K.K.) 16.03.1983**

(73) Proprietor: **TOA NENRYO KOGYO KABUSHIKI KAISHA**
**1-1 Hitotsubashi, 1-Chome Chiyoda-Ku Tokyo 100(JP)**

(72) Inventor: **SAKURADA, Satoshi**
**745-17, Sashiougi Omiya-shi**
**Saitama 330(JP)**
Inventor: **TAGAYA, Nobuaki**
**2735-4, Oaza Kasahata Kawagoe-shi**
**Saitama 350(JP)**
Inventor: **MIURA, Tadashi**
**18, Kuboinari 4-chome Iruma-shi**
**Saitama 358(JP)**
Inventor: **MAESHIMA, Tsugio**
**1428-34, Oaza Suneori Tsurugashimacho Iruma-gun**
**Saitama 350-02(JP)**
Inventor: **HASHIMOTO, Takao**
**1902-5, Oaza Kamekubo Ooimachi, Iruma-gun**
**Saitama 354(JP)**

(74) Representative: **Diamond, Bryan Clive et al**
**Gee & Co., Chancery House, Chancery Lane London WC2A 1QU(GB)**

EP 0 152 485 B1

**Description**

This invention relates to binderless zeolites and more particularly, to TSZ crystalline aluminosilicates having controlled secondary pore formed among crystal grains, production processes thereof and processes using the same as catalysts.

Crystalline aluminosilicates are generally known as zeolites, and both natural and synthetic products are hydrated aluminosilicates having a crystal structure composed fundamentally of three-dimensional frameworks; one of which consists of $SiO_4$ tetrahedra, each tetrahedron formed by coordinating four oxygen atoms at the apexes of a tetrahedron around the central silicon (Si) atom and the other three-dimensional framework consists of $A\ell O_4$ tetrahedra, each tetrahedron formed by replacing the silicon atom in above $SiO_4$ tetrahedron with an aluminum ($A\ell$) atom.

It is known that the $SiO_4$ tetrahedra and the $A\ell O_4$ tetrahedra constitute basic units consisting of 4-, 5-, 6-, 8-, or 12-membered units formed from 4, 5, 6, 8, or 12 joined tetrahedra or basic units consisting of double rings formed from two such 4-, 5-, 6-, 8-, or 12-membered rings, and that the framework of a crystalline aluminosilicate is determined by interconnection of these basic units.

There are certain cavities in the above framework structure, and their apertures are formed with 6-, 8-, 10- or 12-membered rings. Such cavities have a uniform diameter so that molecules smaller than a certain size are adsorbed into them while larger ones can not get into them. Hence, such crystalline aluminosilicates are known as molecular sieves according to their functions, and used as adsorbents, catalysts for chemical reacitons or catalyst carriers in a wide variety of chemical processes.

In recent years, their application as a combination of both a molecular sieve and a catalyst have been energetically studied in a variety of the fields of chemical reactions. These are the so-called molecular shape-selective reaction catalysts and, as shown in the classification made by S.M.Csicsery according to their functions, they are divided into the following three types: (1) catalysts whose active sites can be approached only by special reactants, (2) catalysts in which, among the reactants which have reacted at the active sites, only those having special shapes can leave the reaction sites, and (3) catalysts in which, although individual molecules can freely enter or leave the reaction sites for a bimolecular reaction, they can not react owing to their large transition state ( "Zeolite Chemistry and Catalysis" ACS Monograph 171, ACS, Washington D.C., 1976, p.680).

This classification is made only on the basis of catalytic reactions taking place in the cavities of a crystalline aluminosilicate. For catalytic reactions taking place at active sites on or near the external surface of the crystal, all reactions having low-activation energy can take place — different from the above mentioned catalytic molecular shape selective reactions. Therefore the selectivity of the reactions is lowered.

Accordingly, in order to control the nonselective reactions taking place on or near the external surface of the crystal, there has been proposed a method in which the active sites are buried by coating the crystal surface with a compound and a method in which the solid acidity of the active sites is controlled using some compounds having different solid acidity or alkalinity — such as silicone compounds, phosphorus compounds, magnesium compounds.

On the other hand, a method is also known in which the ratio of the number of active sites (the number of active sites in the crystals, having molecular shape selectivity to that of active sites on or near the surface of crystal, having no shape selectivity) is controlled by controlling the crystal size. For example, when the crystal size is increased, the proportion of the active sites in the crystal increases relatively, and the shape selectivity is heightened.

According to this method, however, the access and/or contact of reactants to and/or with active sites are limited relatively which results in lowered overall reaction activity. Conversely, when the crystal size is decreased, the proportion of the active sites on or near the crystal surface increases relatively and the reaction activity is heightened because of a relative increase in the chance for the reactants to approach or contact active sites, though the shape selectivity is lowered.

The electrical charge of the aluminum-containing tetrahedron of a crystalline sodium aluminosilicate can be balanced by holding sodium cations within the crystal. It is a well-known theory that these cations can bet ion-exchanged by a variety of methods to form a hydrogen-type or a metal-ion exchanged type of crystalline aluminosilicate, which functions as a solid acid catalyst.

In natural crystalline aluminosilicates, the cations are Group I or II metals of the periodic tablet of the elements, especially sodium, potassium, calcium, magnesium and strontium. Also in synthetic crystalline aluminosilicates, the above metal cations are used, but the use of organic nitrogen cations, for example, quarternary alkylammonium ions such as tetraalkylammonium ions, in addition to these metal cations, has recently been proposed.

2

For the synthesis of a crystalline aluminosilicate having a high silica/alumina ratio, it has been thought essential to use a nitrogen-containing organic compound as mentioned above as an alkali metal source. The use of the nitrogen-containing organic compounds, however, has disadvantages in that the material cost is high and that the production process is complicated because, in order to employ the obtained synthetic aluminosilicate as a catalyst, it is necessary to remove the nitrogen-containing compounds contained in the product by calcination at high temperatures.

Moreover, in conventional production processes using the above-mentioned tetraalkylammonium compounds or amine compounds such as $C_2$ to $C_{10}$ primary amines, there has been a problem of operation safety because of the latent toxicity of the organic compounds or various dangers accompanying their decomposition or the like encountered in the synthesis, drying and calcination processes.

Furthermore, although it has been proposed to use oxygen containing organic compounds or sulfur containing compounds, these methods can not solve the problems encountered in using the nitrogen-containing organic compounds.

Recently, these problems were partially solved by an invention disclosed in Japan Patent Application No. 143396/1981 (filed September 11, 1981), in which a crystalline aluminosilicate having characteristic crystal structure characterized by an X-ray diffraction pattern had been obtained.

It is a crystalline aluminosilicate having a chemical composition in terms of a molar ratio of oxides of

$$0.8 \sim 1.5 \ M_2/nO \cdot A\ell_2O_3 \cdot 10 \sim 100 \ SiO_2 \cdot ZH_2O,$$

wherein M is a metal cation, n is the valence of the metal, and Z is 0 to 40, and having the powder X-ray diffraction pattern showing at least the interplanar spacings, i.e., d-spacings, shown in Table 1.

Table 1

| Interplanar spacings: d (Å) | relative intensity (I/Io) |
|---|---|
| 1 1. 2 ± 0. 2 | S. |
| 1 0. 1 ± 0. 2 | S. |
| 7. 5 ± 0. 1 5 | W. |
| 6. 0 3 ± 0. 1 | M. |
| 3. 8 6 ± 0. 0 5 | V.S. |
| 3. 8 2 ± 0. 0 5 | S. |
| 3. 7 6 ± 0. 0 5 | S. |
| 3. 7 2 ± 0. 0 5 | S. |
| 3. 6 4 ± 0. 0 5 | S. |

The aluminosilicate having a crystal structure characterized by the above X-ray diffraction pattern was designated as TSZ.

In Table 1, the relative intensities are given in terms of the symbols: V.S. = very strong. S. = strong, M. = medium, W. = weak and V.W. = very weak.

From another powder X-ray diffractiometric analysis, it was concluded that TSZ belongs crystallographically to the monoclinic system.

On the other hand, when a zeolite catalyst is applied to an industrial process such as a fluidized bed of gas/oil feedstock or a fluidized operation, for example, in catalytic cracking, the zeolite is supplied in the form of fine particles.

It is desirable to increase the surface area of a catalytically active zeolite as much as possible in view of the fact that only the external surfaces of catalyst particles can be utilized almost exclusively, because gas-phase reactions are usually conducted at high space velocities, and the diffusion from the catalyst surface is limited in liquid phase reactions of a heavy oil (US-A-3,966,644 shows that this diffusion limit is about 1/120 in.). Although it can be improved by reducing the diameters of the catalyst particles, the particles are lowered in strength and collapsed. Therefore, the improvement of catalyst performances by this method is limited, and a zeolite catalyst has been applied to industrial processes, after it was molded into pellets by using a suitable amorphous binder. When this method is applied, however, the space velocity of the reactants must be lowered because the utilization rate of the zeolite is lowered, which inevitably lowers the productivity, and in addition, there is a drawback that the zeolite is poisoned as a result of the movement of the alkali, alkaline earth metal or the like contained in the binder into the zeolite. Further, since such pellet-form catalysts are prepared by a process consisting of molding, by compression, a zeolite together with amorphous binder, the binder penetrates into the so-called secondary pores present among the zeolite crystals, and therefore neither the quantity nor distribution of secondary pores could be controlled, although the physical strength is increased.

Further, the above binder has a limitation in that it must be thermally stable and capable of forming paths for gas or liquid reactants passing into the zeolite crystals.

A honeycomb-like solid crystal prepared by coating a conventional base with a zeolite crystal was proposed as a zeolite catalyst excellent in industrial applications (see, for example, British Patent No. 1,441,443 and U.S. Pat. Nos. 3,730,910, 3,468,815, 3,244,643 and 3,697,446), — but in none of these is the production easy, and the catalytic activities, physical strengths and continuance of catalyst activity were inadequate.

According to U.S. Pat. No. 3,119,660, previously formed metakaolin, either alone or in admixture with zeolite A, is reacted with an alkali solution to form 100% zeolite A, or a soluble silica source is added to the reaction mixture to form zeolite X or zeolite Y which is used as a constituent of pellets or the like.

Further, a catalyst composition is known which is prepared by burying a zeolite in a porous matrix and which can provide paths into the crystals while minimizing loss due to abrasion during the operation of the active zeolite crystal (Japan Patent Application OPI No. 133489/1979).

However, in none of above processes is the production easy and no consideration is given to the so-called secondary pores present among crystals. The secondary pores are formed, for example, when a crystalline powder is molded. Therefore, if these pores can be kept effective without decreasing pellet strength, the reactants can easily move from one crystal to another, and in addition, the area of the crystal surface having a catalytic activity is increased practically, with a consequent improvement in the catalytic activity of the pellets. Such a molded zeolite with secondary pores is referred to as "a binderless zeolite".

Accordingly, it is a first object of this invention to provide a catalyst containing TSZ zeolite having effective secondary pores and having a special form excellent in catalytic activity.

It is a second object of this invention to provide a binderless TSZ zeolite excellent as a catalyst used in selectively cracking of n-paraffinic hydrocarbons.

It is a third object of this invention to provide a binderless TSZ zeolite excellent as a catalyst for alkylation of aromatics with alkylating agents such as alcohols or olefins.

It is a fourth object of this invention to provide a process for producing such binderless TSZ zeolites.

These objects have been achieved by subjecting a solid obtained by molding a mixture of a previously synthesized crystalline aluminosilicate with a silica/alumina gel to hydrothermal treatment to crystallize under crystallization conditions.

According to the present invention we provide a process for the production of binderless TSZ zeolite catalyst in the form of a shaped product whereof at least 90% of said zeolite catalyst is a crystalline aluminosilicate, characterized by molding a mixture obtained by mixing (1) 30 to 70 weight % of TSZ crystalline aluminosilicate, component (1) being produced from an aqueous reaction mixture comprising:

| | |
|---|---|
| $SiO_2/Al_2O_3$ : | 10 - 130 mols |
| $Na_2O/SiO_2$ : | 0.01 - 0.5 mols |
| $(Na_2O + M_{2/n}O)/SiO_2$ : | 0.03 - 0.3 mols and |
| $H_2O/(Na_2O + M_{2/n}O)$ : | 150 - 880 mols |

where M is a Group I or II metal cation of the periodic table of the elements, and n is the valence of the metal; with (2) 70 to 30 weight % of a silica/alumina gel having an alumina content of 2 to 10 weight %, wherein the molar ratios of $SiO_2/Al_2O_3$ in components (1) and (2) are nearly equal, and subjecting the resultant molding to hydrothermal treatment which is carried out on an aqueous solution in the presence of a sodium salt in a concentration ranging from 1.5 weight % to the solubility limit until at least 90% of the molding is crystalline aluminosilicate.

These objects have been achieved by subjecting a solid, obtained by molding a mixture of a previously synthesized TSZ crystalline aluminosilicate with a silica/alumina gel to hydrothermal treatment to crystallize under crystallization conditions.

As the previously synthesized crystalline aluminosilicate used in this invention, TSZ crystalline aluminosilicate is used. The crystalline aluminosilicate can be in the state as it is synthesised.

The TSZ crystalline aluminosilicate used in this invention is characterized by an X-ray diffraction pattern obtained by a standard powder X-ray diffractiometric analysis. The crystal structure of the TSZ crystalline aluminosilicate differs remarkably from those heretofore proposed crystalline zeolites in that the diffraction line of $2\theta = 14.7$ (d = 6.03 Å) is a singlet, and that the two diffraction lines of $2\theta = 23$ (d = 3.86 Å) and $2\theta = 23.3$ (d = 3.82 Å) are clearly separated from each other. The lattice spacing of this characteristic X-ray diffraction pattern does not vary markedly even when the substituted cation of the synthesized silicate is exchanged, especially when changed into a H form, or when the $SiO_2/Al_2O_3$ ratio is

changed.

The TSZ crystalline aluminosilicate used in this invention which is still in a state as it is synthesized has a preferable composition, in terms of a molar ratio of oxides, of

$0.8 \sim 1.5$ $Na_2O.Al_2O_3$ $.25 \sim 80$ $SiO_2$ $.0 \sim 40$ $H_2O$,

and it is possible in this case to replace at least part of the metallic cations (which are present when TSZ is synthesized) by ion exchange or a like treatment. This ion exchange can be carried out by using a Group II to VIII metal of the periodic table of the elements, hydrogen ion from acids, or ammonium ion. As far as the $SiO_2/Al_2O_3$ ratio falls within the range of 25 to 80, the crystal structure does not change and the hydrogen-form of TSZ is also monoclinic.

The crystalline aluminosilicate which is able to be used in this invention can be produced generally by preparing an aqueous reaction mixture consisting essentially of inorganic reaction materials, prepared by using $SiO_2$ as a silicon source and $Al_2O_3$ as an aluminum source in the aforesaid ratios and adding a suitable alkali source and water in the above defined ratios, then heating and holding the aqueous reaction mixture at a crystallization temperature until crystals are formed. Such production conditions can be realized, for example by maintaining at an autogenous pressure and a temperature of about $120\,°C$ to about $230\,°C$ for about 10 hours to 10 days.

The TSZ crystalline aluminosilicate is produced from an aqueous reaction mixture consisting substantially of inorganic reaction materials comprising a silica source, an alumina source, an alkali source, water and a neutral salt of an alkali metal, and the composition of said aqueous reaction mixture in terms of a molar ratio of oxides is as follows:

| | |
|---|---|
| $SiO_2/Al_2O_3$ : | $10 \sim 130$ |
| $Na_2O/SiO_2$ : | $0.01 \sim 0.5$ |
| $(Na_2O + M_{2/n}O)/SiO_2$ : | $0.03 \sim 0.3$ |
| $H_2O/(Na_2O + M_{2/n}O)$ : | $150 \sim 800$ |
| $X^-/SiO_2$ : | $0.01 \sim 20$ |

wherein H is a Group I or II metal cation of the periodic table of the elements, and preferably selected from lithium, sodium, barium, calcium and strontium, and n is the valence of the metal.

The $M_{2/n}O$ and the $Na_2O$ are both in a free state and generally take the form of hydroxides, or extremely weak acid salts which are effective in the synthesis of zeolites, for example, aluminates, silicates, etc. Further, the free $Na_2O$ can be controlled by the addition of sulfuric acid, hydrochloric acid, nitric acid or the like.

In this invention it is possible to crystallize a silica/alumina gel which is amorphous in its initial state by molding a mixture prepared by mixing the above produced TSZ crystalline aluminosilicate with the silica/alumina gel into pellets, and then subjecting the pellets to the same hydrothermal treatment as in the production of the TSZ crystalline aluminosilicate, whereby the binderless zeolite of this invention can be obtained.

Since the silica/alumina gel used as binder in this invention crystallizes by the hydrothermal treatment, its composition is one that allows the ready crystallization, with a $SiO_2/Al_2O_3$ ratio nearly equal to that of the TSZ crystalline aluminosilicate in order to obtain a binderless zeolite having a particularly good catalyst performance.

Although the shape of the solid used in the hydrothermal treatment is not particularly limited in this invention, it is preferably pellet, polylobal body or hollow tube on account of the ease of molding or the utilization efficiency in using it as a catalyst, and with respect to its size, outside diameters of about 1.5 mm are preferred for ease of handling.

The hydrothermal reaction in this invention can be carried out according to the method disclosed in Japanese Patent Application No. 143396/1981.

When the hydrothermal treatment in this invention is carried out, the binder crystallizes and shows the X-ray diffraction pattern of TSZ crystalline aluminosilicate, which has the characteristics obtained when synthesis is carried out without using organic cations, and the distribution of secondary pores is extremely sharp. Although a method of measuring the exact radii of secondary pores in the molded zeolite of this invention is not yet established, it is possible to estimate their average radius by so-called mercury porosimetry. In this invention, the average pore radius is defined as the radius at which the accumulated pore volume corresponds to 1/2 of the total pore volume measured by mercury porosimetry. The size of this pore radius is important from the viewpoint of catalytic activity because it concerns not only the actual surface area of the catalyst but also affects the diffusion velocities of reactant molecules and formed molecules.

The binderless TSZ zeolite obtained in this invention has an excellent crystalline structure as a whole. For example, it is possible to obtain binderless zeolite which has a structure so uniform that it is almost impossible to discriminate it microscopically from the starting material.

In the following figures, "before synthesis" refers to the molded pellet before the final hydrothermal treatment.

Fig. 1 shows powder X-ray diffraction patterns of pellets before and after synthesis of the binderless zeolite in Example 1.

Fig. 2 shows electron-microscopic photographs of pellets (prepared in Example 1) of 5000 magnification. Fig. A is a cross section of a pellet (after molding) before synthesis of the binderless zeolite. Fig. B shows the surface of a pellet after the synthesis of the binderless zeolite. Fig. C is a cross section of a pellet after synthesis of the binderless zeolite.

Fig. 3 is a graph illustrating the secondary pore radius distribution of pellets before and after synthesis of the binderless zeolite obtained in Example 1.

The following examples are provided to illustrate the present invention, but are not to be construed as limiting in any way.

EXAMPLE 1

An aluminum sulfate solution was prepared by dissolving 4 g of aluminum sulfate in 170 g of pure water and further adding 5.7 g of concentrated sulfuric acid (95% by weight) and 18 g of sodium chloride. The obtained solution was mixed under agitation with a mixture of 25 g of water and 63 g of water glass (JIS No. 3 water glass, $Na_2O$ content of 9.5% by weight and $SiO_2$ content of 28.6% by weight) to obtain an aqueous reaction mixture having a composition, in terms of the molar ratio of oxides, of

3.9 $Na_2O$ $\cdot$ $Al_2O_3$ $\cdot$ 50 $SiO_2$ $\cdot$ 2184 $H_2O$. The sodium chloride used in this case as a mineralizer had a molar ratio of $Cl^-$ to $SiO_2$ of 1.02. The aqueous reaction mixture was placed in a SUS autoclave, heated and maintained at 180°C for 20 hours under autogenous pressure. The crystallized solid product was separated by filtration, washed with water and dried at 110°C. 50 g of the TSZ zeolite powder thus obtained was kneaded with 380 g of a silica alumina wet gel (water content of 86.8% by weight). The blend was dried to a moldable consistency, and molded into pellets (outside diameter of about 1.5 mm) through an extruder.

The silica alumina wet gel herein used was prepared by adding aqueous aluminum sulfate solution containing 38.6 g of aluminum sulfate, 32 g of 95% sulfuric acid and 330 g of pure water, and an aqueous solution containing 476.2 g of water glass (JIS No. 3, hereinafter abbreviated simply as No. 3) and 240.4 g of pure water to 943 g of pure water and then filtering the combined solutions.

The pellets were dried at about 110°C for 5 hours. Chemical analysis on a proportion of these pellets revealed that it had a composition of 78.9% by weight of $SiO_2$, 4.48% by weight of $Al_2O_3$, 4.18% by weight of $Na_2O$, and 12.4% by weight of ignition loss (900°C).

The pellets were further calcined at 600°C for about 3 hours. A 50 g portion of these pellets together with 43.2 g of sodium chloride and 619 g of pure water was placed in a stainless steel autoclave and heated at 180°C for 40 hours to crystallize.

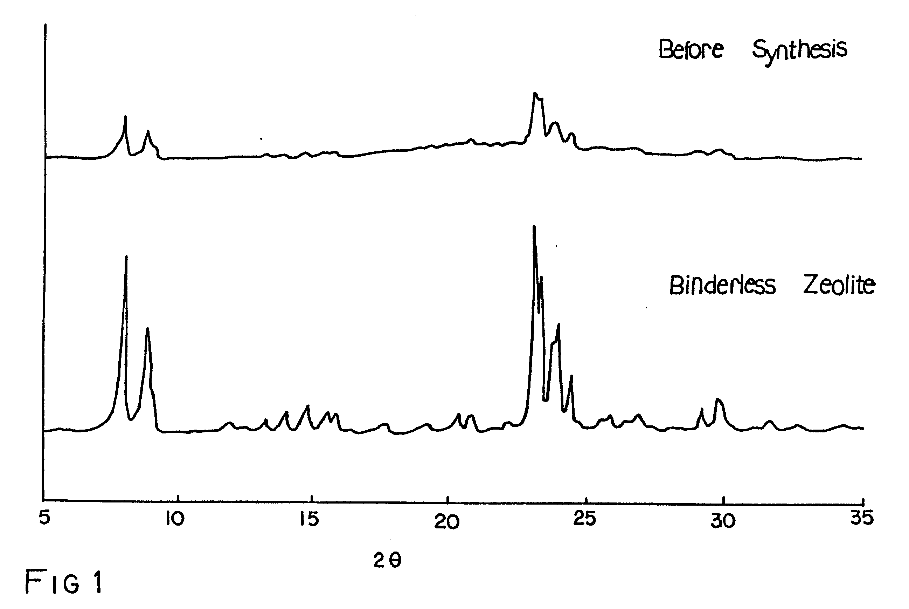

After lowering the temperature, the pellets were withdrawn from the autoclave, washed, dried, and subjected to powder X-ray diffraction analysis to obtain the diffraction pattern of TSZ. Fig. 1 shows the powder X-ray diffraction patterns before and after synthesis.

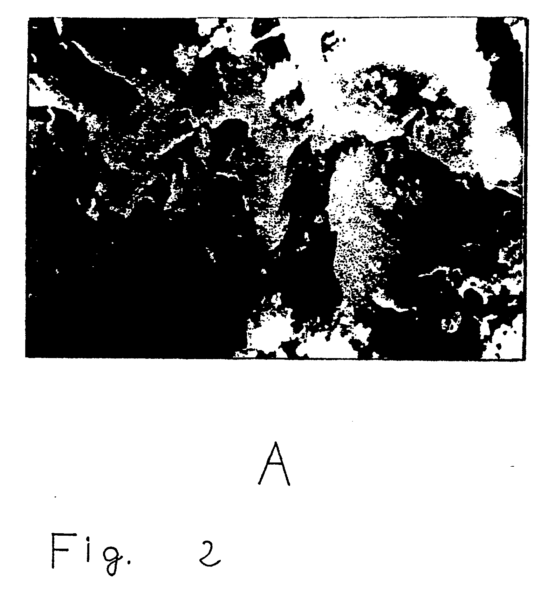

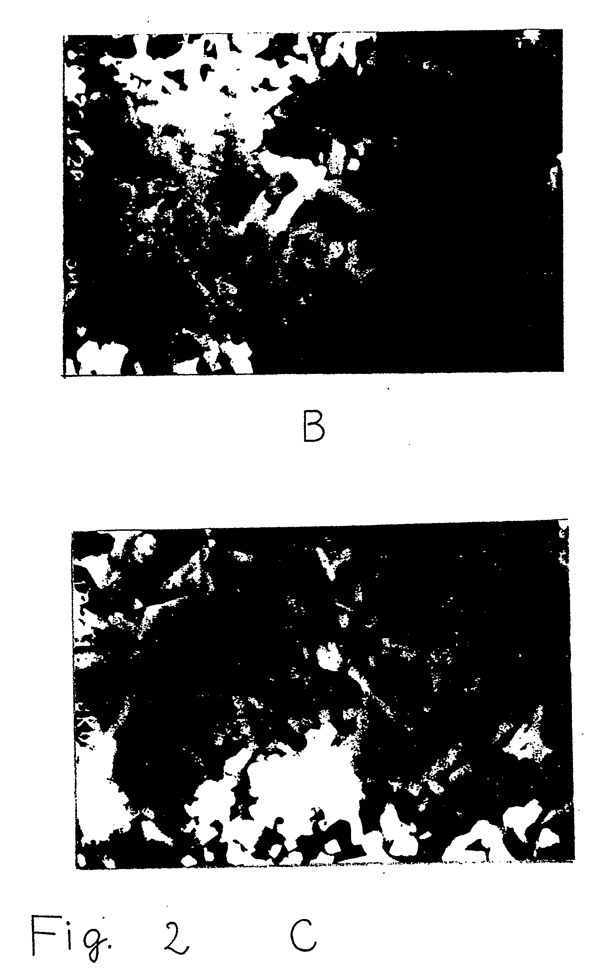

Further, the electron-microscopic photographs showed that the pellets consisted almost entirely of crystalline substances and therefore the silica alumina wet gel had been converted into TSZ. Fig. 2 shows electron-microscopic photographs of the surface and cross section of pellets before and after synthesis.

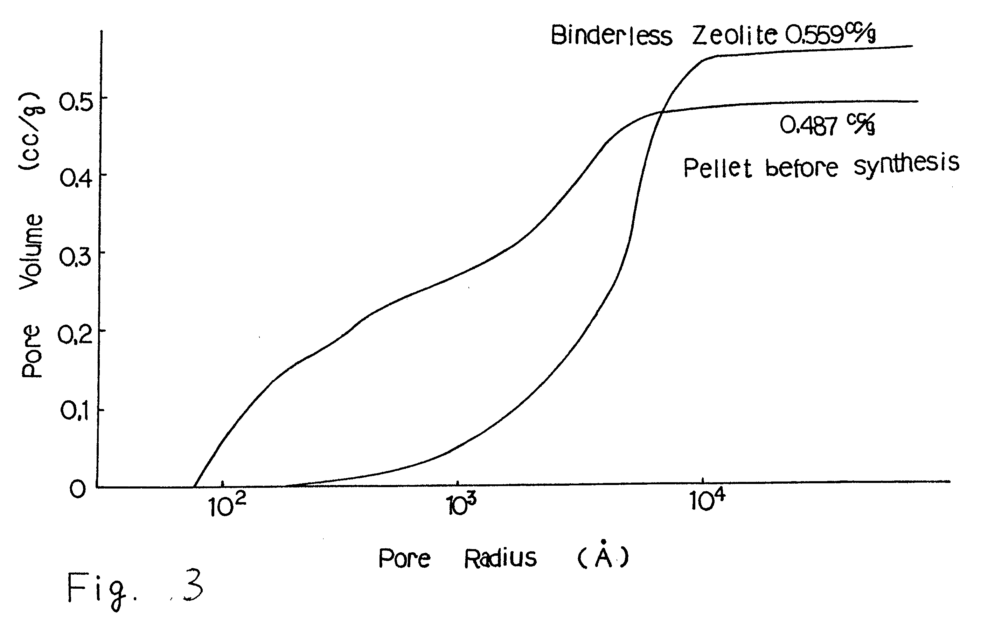

Determination of the pore radius distribution by mercury porosimetry gave a characteristic pore radius distribution curve. Fig. 3 shows pore radius distribution curves before and after synthesis, and shows the total pore volume, as measured by mercury porosimetry, after synthesis was 0.559 cc/g, therefore it was evident that 30% of the total pores were contained within ±20% of the average radius.

The crushing strength of the pellets after drying was 1.5 kg/3 mm, which was a strength sufficient to withstand practical use.

EXAMPLES 2 to 7

Example 1 was repeated (with calcination at 600°C) except that the crystallisation was at 185°C for 44 hours, and that the sodium chloride was omitted in 3 samples whilst sodium hydroxide or TMAOH as cation sources were added in some samples.

The results were shown in Table 2, and show the importance of the cation sources.

## Table 2

| Example | 2 | 3 | 4 | 5 | 6 | 7 |
|---|---|---|---|---|---|---|
| Extrudates (g) | 10.0 | 10.0 | 10.0 | 10.0 | 10.0 | 10.0 |
| NaCl (g) | ---- | 9.6 | ---- | 9.6 | ---- | 9.6 |
| $Na_2 SO_4$ (g) | ---- | ---- | ---- | ---- | 9.6 | ---- |
| NaOH (g) | ---- | ---- | 0.2 | 0.2 | 0.2 | ---- |
| *TMAOH (g) | ---- | ---- | ---- | ---- | ---- | 1.8 |
| $H_2O$ (g) | 137.5 | 137.5 | 137.5 | 137.5 | 137.5 | 137.5 |
| Crystallinity | Bad | Good | Moderate | Good | Good | Bad |

\* TMAOH refers to 30 % of aqueous solution of tetramethylammonium hydroxide.

EXAMPLES 8 to 14

The procedure of Examples 2 to 7 was repeated, with variation in the addition of NaCl and NaOH. The results shown in Table 3 demonstrate that the addition of NaCl is extremely important in this invention.

## Table 3

| Example | 8 | 9 | 10 | 11 | 12 | 13 | 14 |
|---|---|---|---|---|---|---|---|
| Moldings (g) | 10.0 | 10.0 | 10.0 | 10.0 | 10.0 | 10.0 | 10.0 |
| NaCl (g) | ---- | 1.2 | 4.8 | 2.4 | 4.8 | 9.6 | ---- |
| NaOH (g) | ---- | ---- | ---- | 0.2 | 0.2 | 0.2 | 0.2 |
| $H_2O$ (g) | 137.5 | 137.5 | 137.5 | 137.5 | 137.5 | 137.5 | 137.5 |
| Crystallinity | Bad | Moderate | Good | Good | Good | Good | Moderate |

Example 15

30 g of the sodium-form TSZ catalyst obtained in Example 1 was subjected to an ion exchange treatment four times at 80 °C (the treating time for each cycle being 1.5 hours) by using a 5 wt.% ammonium chloride solution in an amount of 15 mℓ per 1g of TSZ. Then, the ion-exchanged product was washed with water, dried at 100 °C and calcined in air at 550 °C for 3 hours to obtain H-TSZ (hydrogen form-TSZ) catalyst. This H-TSZ contained 0.02% by weight of $Na_2O$.

This catalyst was pulverized and classified to a size of ASTM 60/80 mesh. A 0.2 g portion of the powder was placed in a glass flow reactor.

A solution prepared by mixing n-hexane with 3-methylpenthane in a weight ratio of 1: 1 was cooled to 10 °C and passed together with nitrogen as a carrier gas so that the weight hourly space velocity (W/H/W) could be 1.36. Table 4 shows the reaction rates at 300, 350 and 400 °C after 0.5 hour.

7

Comparative Example 1

The TSZ zeolite prepared according to the method of Example 1 was converted into H (hydrogen) form TSZ in the same manner as in Example 15. This H-TSZ contained 0.02% by weight of $Na_2O$.

The wet gel used in Example 1 of the present application was thoroughly washed with a 1.5% ammonium carbonate solution. This wet gel was dried at 110 °C for 5 hours, and subjected to chemical analysis. It was found that the $Na_2O$ content was 0.05% by weight.

The gel thus obtained was mixed with the zeolite so that the weight ratio of the former to the latter could be in the range of 70:30 after calcination at 600 °C for 3 hours, and molded. The extrudate was calcined at 600 °C for 3 hours, pulverized in the same way as in Example 15, and classified to a size of ASTM 60/80 mesh. A 0.2 g portion of this powder was placed in a glass flow reactor.

The reaction was carried out in the same manner as in Example 15 of the present application, and the results shown in Tables 4 and 5 were obtained.

These results demonstrated that the binderless zeolite catalyst of this invention was particularly excellent in respect of reaction rates and yields of aromatics.

The weight hourly space velocity (W/H/W) in this case was adjusted to 0.95 (W/H/W) so as to coincide with the case of Example 15.

8

## Table 4

| Rate constant | k (hr⁻¹) | | |
|---|---|---|---|
| Temperature (℃) | 300 | 350 | 400 |
| Example 15 | | | |
| n-hexane | 0.40 | 1.22 | 2.58 |
| 3-methylpentane | 0.04 | 0.29 | 0.96 |
| Comparative Example 1 | | | |
| n-hexane | 0.37 | 0.78 | 1.42 |
| 3-methylpentane | 0.05 | 0.21 | 0.68 |

## Table 5

| Temperature (℃) | | 300 | 350 | 400 |
|---|---|---|---|---|
| Example 15 | | | | |
| Product | $H_2$ | 0.39 | 1.12 | 1.37 |
| composition | P | 82.1 | 63.0 | 54.8 |
| (% by weight) | | | | |
| | O | 15.8 | 25.4 | 24.6 |
| | Ar | 1.7 | 10.5 | 19.3 |
| Comparative Example 1 | | | | |
| Product | $H_2$ | 0.40 | 0.66 | 0.57 |
| composition | P | 82.4 | 71.2 | 63.3 |
| (% by weight) | | | | |
| | O | 15.0 | 22.3 | 26.1 |
| | Ar | 2.2 | 5.8 | 10.0 |

P: Paraffins, O: Olefins, Ar: Aromatics

Example 16

The binderless catalyst (H-TSZ) obtained in Example 15 was pulverized and classified to a size of ASTM 25/60 mesh. A 3g portion of this was placed in a glass flow reactor, through which a starting material comprising a 1, 2, 4-trimethylbenzene/methanol mixture (molar ratio of 1/2) as shown in Table 6 was passed at 325°C. Table 7 shows conversions of trimethylbenzene, selectivities for $C_{10}$ aromatic products and isomer distributions of $C_{10}$ aromatic products which were measured 4 hours after the start of the reaction.

Comparative Example 2

The binder and the H-TSZ which were obtained in Comparative Example 1 were pulverized together and the powder was classified to a size of ASTM 25/60 mesh. A 3g portion of this was placed in a glass flow reactor, through which a starting material comprising a 1, 2, 4-trimethylbenzene/methanol mixture (molar ratio of 1/2) as shown in Table 6 was passed at 325 °C. While the weight hourly space velocity in Example 16 was 3.2 W/H/W, that in this Example was set at 2.3 W/H/W so that both of the space velocities of the materials over the zeolite catalyst would be equal. Table 7 shows the results together with those of Example 16.

These results demonstrated that the binderless zeolite catalyst of this invention was extremely excellent in selectivities for $C_{10}$ aromatic products.

## Table 6

| Reaction Conditions | Example 16 | Comparative Example 2 |
|---|---|---|
| | | Comparative |
| Catalyst obtained in | Example 15 | Example 1 |
| Material: | | |
| TMB/methanol (mol/mol) | 1/2 | 1/2 |
| Temperature (°C) | 325 | 325 |
| Pressure | Atmospheric | Atmospheric |
| Weight hourly space velocity (W/H/W) | 3.2 | 2.3 |
| Conversion: TMB (%) | 22.4 | 19.3 |
| Yield of aromatic product (mol % based on TMB) | 100.3 | 100.1 |

Note: TMB refers to 1, 2, 4-trimethylbenzene

## Table 7

| Results of reaction | | Example 16 | Comparative Example 2 |
|---|---|---|---|
| Selectivity for | $C_6$ | 0 | 0.3 |
| aromatic product | $C_7$ | 0.2 | 1.6 |
| (molar %) | $C_8$ | 6.8 | 13.7 |
| | $C_9$ | 6.4 | 10.9 |
| | $C_{10}$ | 84.4 | 73.4 |
| | $C_{11}^+$ | 2.2 | 0.1 |
| Isomer | 1, 2, 4, 5-TeMB | 88.6 | 88.6 |
| distribution of | 1, 2, 3, 5-TeMB | 4.6 | 5.3 |
| $C_{11}$ aromatic | 1, 2, 3, 4-TeMB | 5.6 | 3.9 |
| products | Others | 1.2 | 2.2 |

Note: TeMB refers to tetramethylbenzene

Example 17

The binderless catalyst (H-TSZ) obtained in Example 15 was pulverized and classified to a size of 0.71-0.25mm (ASTM 25/60 mesh). A 1g portion of this H-TSZ was placed in a glass flow reactor. A toluene/ethylene/hydrogen mixture (molar ratio of 5/1/5)was passed over the catalyst at atmospheric pressure and a weight hourly space velocity of toluene of 6.5 W/H/W. The liquid product recovered during a period from 4.5 to 5.0 hours after the start of the reaction was analyzed gas-chromatographically to determine the conversion of toluene, and the isomer distribution of ethyltoluene products.
Table 6 shows the results.

Comparative Example 3

The H-TSZ obtained in Comparative Example 1 was blended with an alumina binder ($Na_2O$ content of 0.005% by weight) prepared from aluminum sulfate and sodium aluminate used in amounts to provide a weight ratio of the former to the latter of 70/30 after calcination at 600 °C for 3 hours, and then molded. After drying at 110 °C, the extrudate was calcined at 660 °C for 3 hours to form a binder-containing catalyst. This was classified into a size of ASTM 26/60 mesh and used in the reaction similar to that in Example 17. In this case, the weight hourly space velocity of toluene over the zeolite was adjusted to 4.6 W/H/W so as to coincide with the case of Example 17.
The sample recovered 4.5 to 5.0 hours after the start of the reaction was analyzed to obtain the results shown in Table 8.

Table 8

|  | Example 17 | Comparative Example 3 |
| --- | --- | --- |
| Raw Material: | | |
| toluene/ethylene/hydrogen (molar ratio) | 5/1/5 | 5/1/5 |
| Temperature (°C) | 350 | 350 |
| Conversion of toluene (% by weight) | 19.22 | 18.76 |
| Isomer distribution of ethyltoluene (%) | | |
| p- | 30.19 | 28.18 |
| m- | 61.23 | 63.49 |
| o- | 8.58 | 8.33 |

These results demonstrated that the binderless zeolite of this invention was excellent in conversion of toluene and selectivity for p-ethyltoluene formation as compared with the conventional catalyst.

POSSIBILITY OF INDUSTRIAL USES

As is different from powder forms the binderless zeolite catalyst obtained in this invention can be extremely easily handled in the subsequent operations such as washing, conversion into hydrogen form and ion exchange of active metal species. The binderless zeolite of this invention thus obtained is not only excellent in activity because of controlled secondary pores but also excellent in ability to retain its activity, and exhibits an excellent performance as a catalyst for the selective cracking of n-paraffinic hydrocarbons or the alkylation of aromatics with alkylating agents such as alcohols or olefins.

11

## Claims

1. A process for the production of binderless zeolite catalyst in the form of a shaped product whereof at least 90% of said zeolite catalyst is a TSZ crystalline aluminosilicate, characterized by molding a mixture obtained by mixing (1) 30 to 70 weight % of TSZ crystalline aluminosilicate, component (1) being produced from an aqueous reaction mixture comprising:

   $SiO_2/Al_2O_3$ :           10 - 130 mols
   $Na_2O/SiO_2$ :           0.01 - 0.5 mols
   $(Na_2O + M_{2/n}O)/SiO_2$ :     0.03 - 0.3 mols and
   $H_2O/(Na_2O + M_{2/n}O)$ :     150 - 800 mols

   where M is a Group I or II metal cation of the periodic table of the elements, and n is the valence of the metal; with (2) 70 to 30 weight % of a silica/alumina gel having an alumina content of 2 to 10 weight %, wherein the molar ratios of $SiO_2/Al_2O_3$ in components (1) and (2) are nearly equal, and subjecting the resultant molding to hydrothermal treatment which is carried out on an aqueous solution in the presence of a sodium salt in a concentration ranging from 1.5 weight % to the solubility limit until at least 90% of the molding is crystalline aluminosilicate.

2. Binderless zeolite whereof at least 90% shows the X-ray diffraction pattern of TSZ aluminosilicate as produced by the process claimed in Claim 1.

3. Binderless zeolite as claimed in Claim 2, wherein said shaped product is a pellet, polylobal body or hollow tube.

4. Binderless zeolite as claimed in Claim 2 or 3, wherein said crystalline aluminosilicate is a hydrogen form or a salt with a Group VIII metal ion in the periodic table of the elements.

5. Binderless zeolite as claimed in any one of Claims 2 to 4, wherein said catalyst has a pore radius ranging from 75 to 75,000 Å, as measured by mercury porosimetry, and at least 25% of the pore volume is occupied by pores having pore radii in the range of said average pore radius ±20%.

6. Binderless zeolite as claimed in Claim 5, wherein the total pore volume as measured by mercury porosimetry is at least 0.3 cc/g.

7. A process for selectively cracking a paraffinic hydrocarbon, wherein binderless zeolite, as claimed in any one of Claims 2 to 6, is used as a catalyst.

8. A catalytic process for alkylation of aromatic hydrocarbons with an alkylating agent selected from the alcohols and olefins, wherein binderless zeolite, as claimed in any one of Claims 2to 6, is used.

## Revendications

1. Procédé pour la production d'un catalyseur à base de zéolite sans liant sous la forme d'un produit façonné, dont au moins 90% du catalyseur à base de zéolite est un aluminosilicate cristallin TSZ, caractérisé en ce qu'on moule un mélange obtenu en mélangeant :

   (1) 30 à 70% en poids d'aluminosilicate cristallin TSZ, ce composant (1) étant produit à partir d'un mélange de réaction aqueux contenant :

   $SiO_2/Al_2O_3$ :           10 à 130 moles
   $Na_2O/SiO_2$ :           0,01 à 0,5 mole
   $(Na_2O + M_{2/n}O)/SiO_2$ :     0,03 à 0,3 mole, et
   $H_2O/Na_2O + M_{2/n}O)$ :     150 à 800 moles,

   où M est un cation métallique du groupe I ou II du Tableau périodique des éléments, et n est la valence du métal, avec

   (2) 70 à 30% en poids d'un gel de silice et d'alumine ayant une teneur en alumine de 2 à 10% en poids, les rapports molaires de $SiO_2/Al_2O_3$ dans les composants (1) et (2) étant pratiquement égaux, et en ce qu'on soumet le produit moulé obtenu à un traitement hydrothermique qui est conduit sur une solution aqueuse en la présence d'un sel de sodium en une concentration comprise entre 1,5% en poids jusqu'à la limite de solubilité jusqu'à ce qu'au moins 90% du produit moulé soit de l'aluminosilicate cristallin.

2. Zéolite sans liant, dont au moins 90% présentent le modèle de diffraction aux rayons X d'un aluminosilicate TSZ, comme produit par le procédé revendiqué dans la revendication 1.

3. Zéolite sans liant selon la revendication 2, dans laquelle le produit façonné est une pastille, un corps à plusieurs lobes ou un tube creux.

4. Zéolite sans liant selon la revendication 2 ou la revendication 3, dans laquelle l'aluminosilicate cristallin est une forme hydrogène ou un sel avec un ion métallique du groupe VIII du Tableau périodique des éléments.

5. Zéolite sans liant selon l'une quelconque des revendications 2 à 4, dans laquelle le catalyseur a un rayon de pores compris entre 75 et 75 000 Å, comme mesuré par porosimétrie au mercure, et au moins 25% du volume des pores est occupé par des pores ayant des rayons de pores égaux au rayon de pore moyen ± 20%.

6. Zéolite sans liant selon la revendication 5, dans laquelle le volume total des pores, mesuré par porosimétrie au mercure, est au moins 0,3 cc/g.

7. Procédé pour le craquage sélectif d'un hydrocarbure paraffinique, dans lequel on utilise comme catalyseur une zéolite sans liant, selon l'une quelconque des revendications 2 à 6.

8. Procédé catalytique pour l'alcoylation d'hydrocarbures aromatiques avec un agent alcoylant choisi parmi les alcools et les oléfines, dans lequel on utilise une zéolite sans liant selon l'une quelconque des revendications 2 à 6.

**Patentansprüche**

1. Verfahren zur Herstellung eines binderlosen Zeolith-Katalysators als geformtes Produkt, wobei wenigstens 90 % des Zeolith-Katalysators ein TSZ-kristallines Aluminosilikat ist, gekennzeichnet durch das Verformen einer Mischung aus

1) 30 - 70 Gew.-% von TSZ-kristallinem Aluminosilikat, welches gebildet worden ist aus einer wässrigen Reaktionsmischung umfassend:

| | |
|---|---|
| $SiO_2/Al_2O_3$ : | 10 - 130 mol |
| $Na_2O/SiO_2$ : | o.o1 - o.5 mol |
| $(Na_2O + M_{2/n}O)/SiO_2$ : | 0.03 - 0.3 mol und |
| $H_2O/(Na_2O + M_{2/n}O)$ : | 150 - 800 mol, |

wobei M ein Metallkation der Gruppe I oder II des Periodensystems der Elemente ist und n die Wertigkeit des Metalls angibt,

2) 70 - 30 Gew.-% eines Kieselerde/Tonerde-Gels mit einem Tonerdegehalt von 2 - 10 Gew.-%, wobei die molaren Verhältnisse von $SiO_2/Al_2O_3$ in den Komponenten (1) und (2) nahezu gleich sind, und durch Unterwerfen des resultierenden Formlings unter eine hydrothermale Behandlung, die in einer wässrigen Lösung in Anwesenheit eines Natriumsalzes in einer Konzentration zwischen 1,5 Gew.-% bis zum Löslichkeitslimit durchgeführt wird, bis wenigstens 90 % des Formlings kristallines Aluminosilikat ist.

2. Binderloser Zeolith, wobei wenigstens 90 % ein Röntgenstrahl-Diffraktionsmuster von TSZ Aluminosilikat zeigt, welches nach dem verfahren nach Anspruch 1 hergestellt ist.

3. Binderloser Zeolith nach Anspruch 2, dadurch gekennzeichnet, daß er in Form eines Kügelchens, eines mehrflügligen Körpers oder eines hohlen Rohrs geformt ist.

4. Binderloser Zeolith nach Anspruch 2 oder 3, dadurch gekennzeichnet, daß das kristalline Aluminosilikat eine Hydrogenform oder ein Salz mit einem Metall-Ion aus der Gruppe VIII des Periodensystems der Elemente ist.

5. Binderloser Zeolith nach einem der Ansprüche 2 bis 4, dadurch gekennzeichnet, daß der Katalysator einen Porenradius aufweist, der zwischen 75 und 75.000 A, gemessen durch Quecksilber-Porosimetrie, aufweist und daß wenigstens 25 % des Porenvolumens durch Poren gebildet wird, die Porenradien im

13

Bereich des genannten durchschnittlichen Porenradius ± 20 % besitzen.

6. Binderloser Zeolith nach Anspruch 5, dadurch gekennzeichnet, daß das durch Quecksilberporosimetrie gemessene Gesamtporenvolumen wenigstens 0,3 cc/g beträgt.

7. Verfahren zum selektiven Kracken eines paraffinischen Kohlenwasserstoffs, gekennzeichnet durch die Verwendung eines binderlosen Zeoliths gemäß einem der Ansprüche 2 bis 6 als Katalysator.

8. Katalytisches Verfahren zur Alkylierung aromatischer Kohlenwasserstoffe mit einem Alkylieragens, ausgewählt aus Alkoholen und Olefinen, gekennzeichnet durch Verwendung binderlosen Zeoliths nach einem der Ansprüche 2 bis 6.

Before Synthesis

Binderless Zeolite

5   10   15   20   25   30   35

2θ

FIG 1

EP 0 152 485 B1

A

Fig. 2

B

Fig. 2    C

Fig. 3

EP 0 152 485 B1